Europäisches Patentamt

European Patent Office

Office européen des brevets

(11) Veröffentlichungsnummer: **0 339 541**

**A1**

(12) # EUROPÄISCHE PATENTANMELDUNG

(21) Anmeldenummer: **89107377.7**

(51) Int. Cl.⁴: **A61B 1/26**

(22) Anmeldetag: **24.04.89**

(30) Priorität: **23.04.88 DE 8805398 U**

(43) Veröffentlichungstag der Anmeldung:
**02.11.89 Patentblatt 89/44**

(84) Benannte Vertragsstaaten:
**AT CH DE ES FR IT LI**

(71) Anmelder: **GUSTAV MÜLLER GmbH & Co., KG.**
**Fabrik für chirurgische Instrumente**
**Elsa Brandström-Weg 21**
**D-7200 Tuttlingen(DE)**

(72) Erfinder: **Buhl, Rolf Walter, Dr. med.**
**Parsevalstrasse 16**
**D-5600 Wuppertal 2(DE)**
Erfinder: **Bertz, Birgit Agnes Elly**
**Hünefeldstrasse 54**
**D-5600 Wuppertal 2(DE)**
Erfinder: **Anders, Fridolin**
**Hauptstrasse 42**
**D-7717 Immendingen 4 (Hattingen)(DE)**

(74) Vertreter: **Bierl, Richard, Dr. rer. nat.,**
**Dipl.-Phys.**
**Hauptstrasse 32/I**
**D-7218 Trossingen 1(DE)**

(54) **Gerät für ärztliche und/oder pflegerische Massnahmen.**

(57) Bei einem Gerät für ärztliche und/oder pflegerische Maßnahmen, insbesondere im Bereich von Operationsmethoden, wie der endotrachealen Intubation, im Zusammenhang mit visueller Untersuchung, bzw. im Bereich der Bronchial- und der Mund-Toilette, mit einem Handgriff, an dessen Gehäuse mit Lichtquelle, Energiequelle zu deren Stromversorgung und eine Halterungsvorrichtung für ein Formteil, zB. ein Spatel, mit Lichtkanal und dessen Lichtaustrittsöffnung angeordnet ist, ist der Spatel blattförmig und zungenförmig gestaltet, wobei seine Breite ab der Pharynx der Zunge bis zu ihrem foramen caecum stetig zunimmt, an dieser Stelle mindestens annähernd zwei Drittel der Gaumenbreite einnimmt und von da ab in die gerundete Zungenspitze ausläuft, während das Spatelblatt einen Längsabschnitt mit Lichtkanal (-kanälen) und Lichtleiter aufweist, wobei die Lichtaustrittsöffnung nahe einer fiktiven Tangente an die Unterfläche des Spatelblatts, von der trachea aus angelegt, dh. an der Stelle mit der größten Zungenmuskelmasse, dh. der Stelle der medizinisch optimalen Pressung der Zunge während des Einsatzes des Laryngoskops im annähernd üblichen Abstand vom distalen Ende des Spatelblatts liegt.

FIG. 2

## Gerät für ärztliche und/oder pflegerische Maßnahmen

Die Erfindung betrifft ein Gerät für ärztliche und/oder pflegerische Maßnahmen, insbesondere im Bereich von Operationsmethoden, wie der endotrachealen Intubation in der Anästhesiologie, Intensivtherapie und Notfallmedizin, im Zusammenhang mit visueller Untersuchung von Körperhöhlen und -gängen, bzw. im Bereich der Bronchial- und der Mund-Toilette, sowohl auf dem oralen bzw. dem nasalen Wege.

Das hier in Rede stehende Gerät ist mit einem Handgriff, an dessen Gehäuse, enthaltend eine elektrische Lichtquelle, eine zugehörige Energiequelle zu deren Stromversorgung und einen der Lichtquelle zugewandten Sockel, eine Halterungsvorrichtung für ein darin griffstückseitig rastend schwenkbar gelagertes Formteil, zB. ein Spatel, mit Lichtkanal und dessen Lichtaustrittsöffnung nahe dem distalen zweiten Viertel seiner Länge angeordnet ist, ausgestattet.

Bisherige Laryngoskope weisen seit jeher an dem Spatel eine mehr oder minder hohe Seitenwand auf, die bei Anwendung am Patienten für den Benutzer, zB. dem behandelndem Arzt, auf der linken Seite angebracht ist. An dieser Seitenwand ist bekanntlich entweder bei einem Warmlicht-Laryngoskop eine elektrische Lampe zur Ausleuchtung der Mundhöhle und der trachea aufgebracht, oder bei einem in der neueren Zeit üblicheren Kaltlicht-Laryngoskop ein Fiberglasfaserstrang-Lichtleiter untergebracht, der die Lichtstrahlung einer Glühlampe im Handgriff des Instruments od.dgl. für den gleichen Zweck weiterleitet.

Dieser Aufbau des Instruments hat sich im großen und ganzen an sich bewährt, weil dadurch erstens eine Führung von instabilen weichen endotrachealen Tuben ermöglicht, zweitens das Vorwölben durch das relativ schmale Laryngoskop-Blatt des Spatels in das Sehfeld des Arztes heruntergedrückten Zunge verhindert, und damit das Freihalten des Sichtkanals auf den Kehlkopfeingang ermöglicht wird.

Diese erwünschten Wirkungen haben jedoch mehrere Nachteile, von denen vor allem Behinderungen sehr störend sind, wie zB., daß das Sichtfeld durch die Seitenwand links begrenzt wird, wobei aber aus dem gleichen Grunde der Tubus nach rechts eingeführt werden kann und trotz guter Ausleuchtung der Stimmritze die Ausleuchtung des Mund-Rachenraums nach links begrenzt, ferner der Einsatz gewisser Hilfsinstrumente, wie zB. eine Magill-Zange, in der Bewegungsfreiheit, oder bei notfallmäßigem Gebrauch der Bewegungsspielraum des Fingers, um zB. Erbrochenes aus dem Mundbereich auszuräumen, eingeschränkt wird oder nicht zuletzt eine Begünstigung von Verletzungen im Bereich der Zahnleiste und der Lippen und dadurch verursachte Narkoseeinleitungsschäden oder andere Lesionen infolge der kantigen Form in Kauf genommen werden müssen.

Der neuen Erfindung liegt die Aufgabe zugrunde, das hier in Rede stehende medizinische Instrument mit einer Formgebung auszustatten, bei der die Gefahr einer Behinderung der Sicht auf wesentliche Teile des Mund-und Rachenraums für die Intubation durch Vorwölbung der Zunge, vor allem die Gefahr von Verletzungen nunmehr weniger als bisher besteht und eine leichtere Reinigung und Desinfektion gewährleistet ist, und zwar je nach dem sowohl bei ärztlichen als auch unabhängig davon pflegerischen Maßnahmen. Diese Aufgabe wird bei einem Gerät nach dem Oberbegriff des 1.Anspruchs in Verbindung mit den dort genannten Merkmalen durch die Kombination mit den kennzeichnenden Merkmalen gelöst.

Ausführungsbeispiele sind anhand der Zeichnung in der folgenden Beschreibung abgehandelt, es stellen dar:

Fig. 1: eine perspektivische Außenansicht von hinten auf den Sockel zwischen Spatel und Handgriff eines Ausführungsbeispiels mit Lichtleiter auf der Oberseite des Spatelblatts;

Fig. 2: eine perspektivische Außenansicht von vorn mit Sicht auf die Lichtaustrittsöffnung des Lichtleiters;

Fig. 3: eine Darstellung der Anwendung eines Spatels im Gaumen eines Patienten bei zusammengedrückter Zunge:

a) entnommen aus einer fotografischen Darstellung mit Warmlicht-Laryngoskop;

b) mit einem Kaltlicht-Laryngoskop in der erforderlichen Lage für Intubation mit der hierfür notwendigen erhöhten Lagerung und Reklination des Kopfes einerseits und Anheben des Zungengrunds und der epiglottis durch das Laryngoskop andererseits;

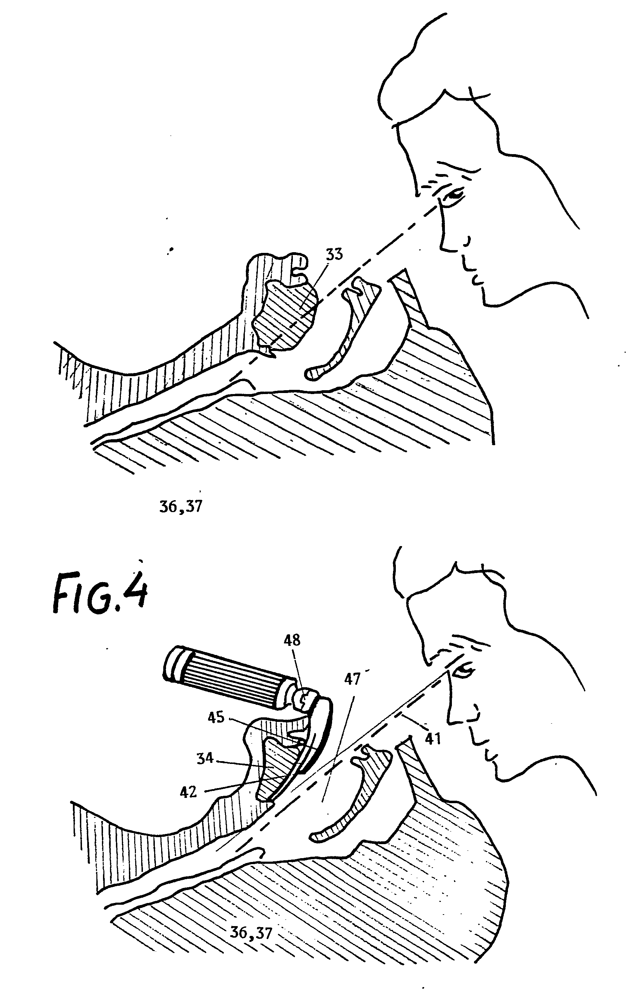

Fig.4: eine fiktive Darstellung des Zungen-Gaumen-trachea-Kehlkopf-Raumes in der Stellung mit nach hinten und nach oben geschwenktem Kopf, wobei eine Tube über die Zunge hinweg in der trachea entlang der gestrichelten Sehlinie geführt gedacht ist;

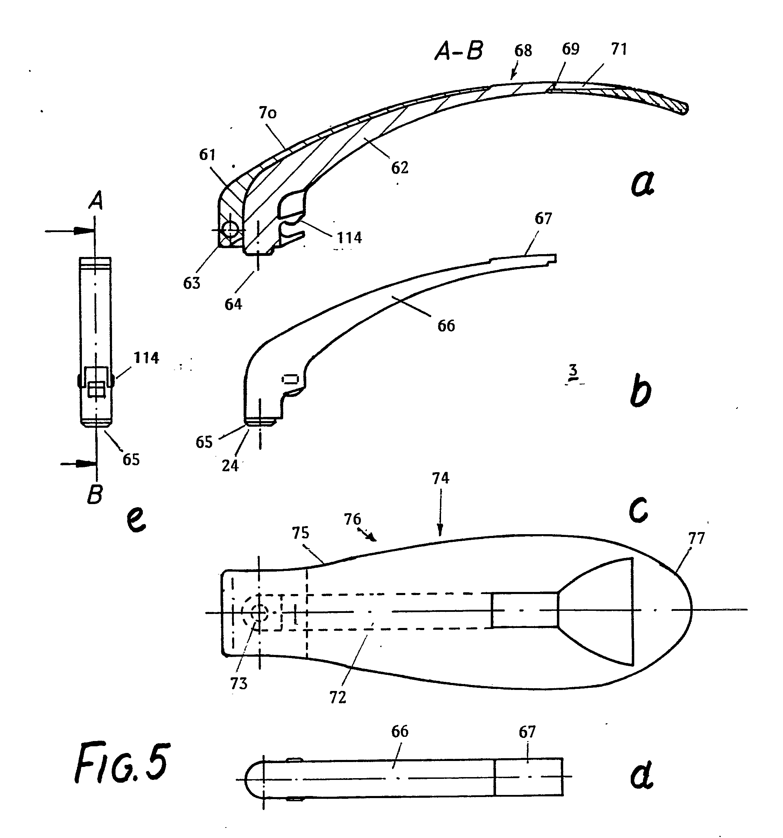

Fig. 5: einen Spatel mit eingesetztem Lichtleiter

a) zusammengesetzt in Gesamtsicht und Schnitt dargestellt, in Einzeldarstellung:

b) in Außen-/Seitenansicht

c) in der Draufsicht des Spatelblatts

d) in der Draufsicht des Lichtleiters

e) in der Seitenansicht;

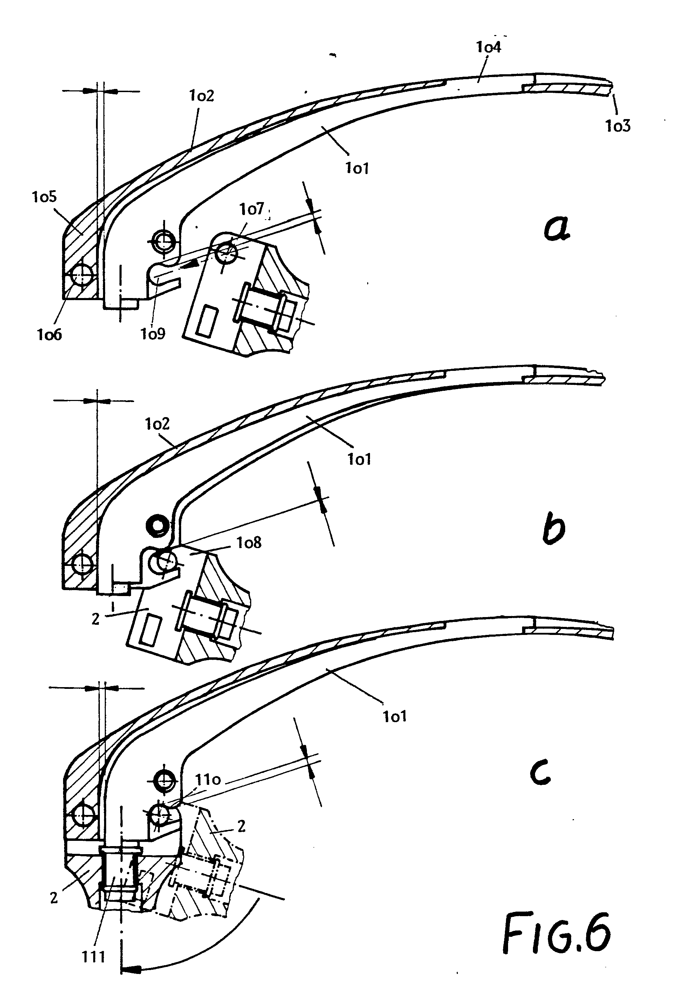

Fig.6: die Gruppe Spatelblatt, Lichtleiter-Einheit und Sockelabschnitt des Handgriffs im Zusammenwirken

a) getrennt

b) in der teilweise eingesteckten Lage von Lagerbolzen und Lagernut

c) die gleiche Kombination mit vollständig eingeschnapptem Lagerzapfen;

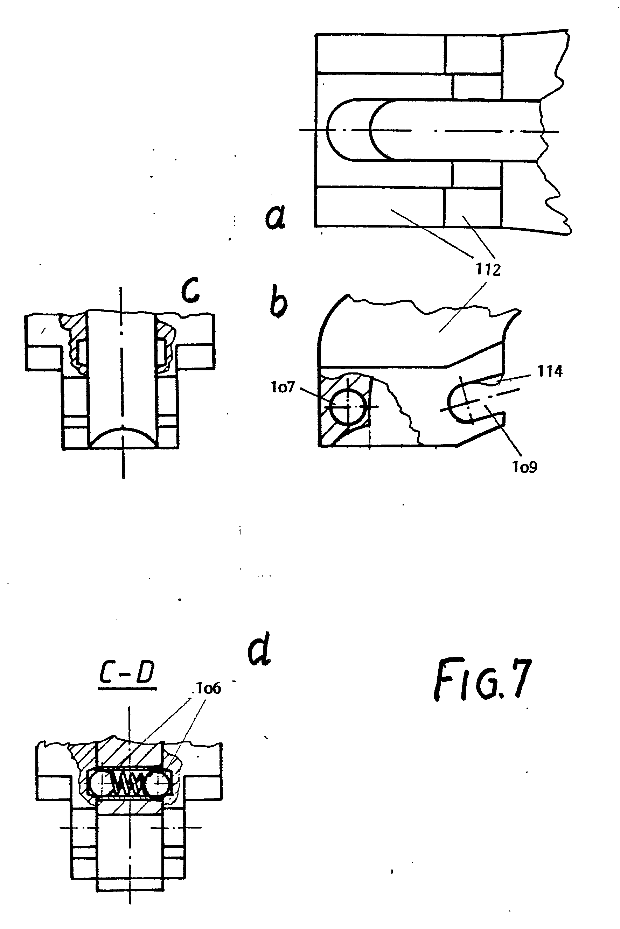

Fig. 7: eine Teildarstellung des Sockels mit der Lagerbohrung und der Nut für den Schwenkbolzen und eine dazu alternative Ausbildung von der Spatel-Lichtleiter-Einheit mit ebenfalls alternativer Schnappvorrichtung für die Rastung

a) bis c) ein Detail des Schwenk-Lagerkopfes

d) ein Detail der Kugelanordnung.

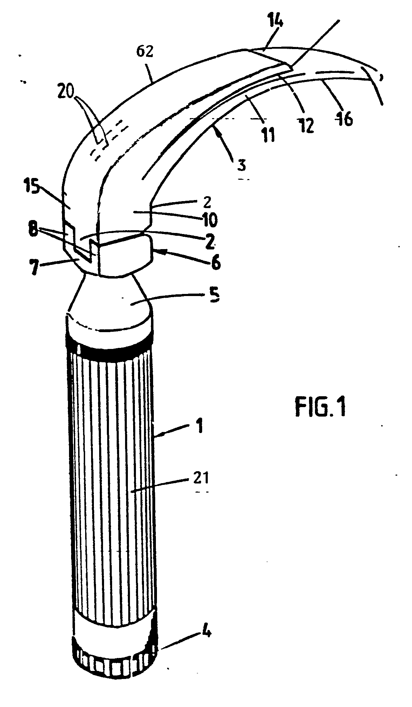

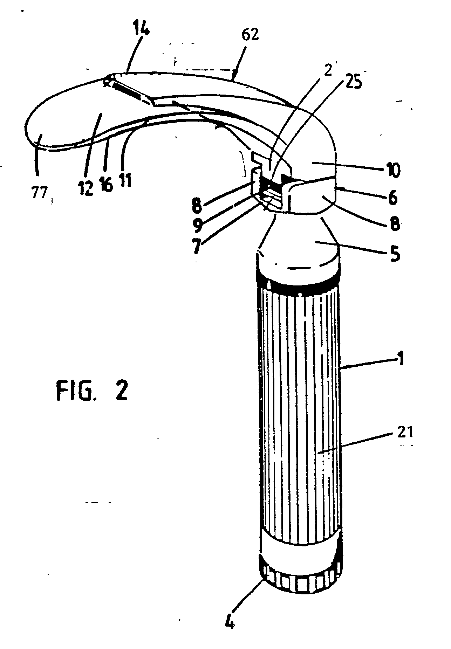

Der Handgriff 1 ist, wie Fig.1 und 2 zeigen, als Rohr 2 ausgebildet, in dessen Innerem - nicht explizit dargestellt - eine als Stromquelle für die ebenfalls dort eingebaute, als Lichtquelle dienende Glühlampe untergebracht ist. Am oberen Ende des Handgriffs 1 ist ein Sockel 5 und mit diesem eine Halterungsvorrichtung 6 angeordnet, in der der Spatel 15 als Formteil 3 mittels Gelenkstück 2 Sockelbacken 8 und einem Zapfensteg 7 mit Einhakachse 9 griffstückseitig aus der dargestellten Betriebslage in die im rechten Winkel hierzu rastend schwenkbar gelagert ist.

An das Fußstück 1o des Spatels 3 schließt sich das Spatelblatt 11 an, das in Längsrichtung zungenförmig gestaltet ist und mindestens einen Lichtkanal in die Unterseite 16 bzw. die Oberfläche der griffseitigen Dreiviertel des Spatelblatts 11 herausgearbeitet enthält. Die Lichtaustrittsöffnung 14 am Ende des Lichtkanals ist als Abschrägung 17 bzw. Abflachung 71 zwecks guten Übergangs in die Außenfläche 12 des Spatelblatts ausgebildet.

Das Fußstück 1o ist einstückig für zwei Schenkel ausgebildet und mit einer Einhakachse in der Einhaköffnung 25 ausgestattet, so daß an ihr ein rastend schnappend hakenförmiger Hebel-Schenkel 13 eingehängt werden kann. Neben der Welle des Zapfenstegs 7 ist der Lichtleiter-Faserstrang in eine nicht dargestellte Abschlußhülse 24 geführt, die in der Halterungsvorrichtung 6 der Lichtquelle gegenüber angeordnet ist. Am anderen Ende ist das Rohr 2 durch einen Schraubdeckel 4 verschlossen, so daß die eingebauten Teile gegen Herausfallen gesichert sind.

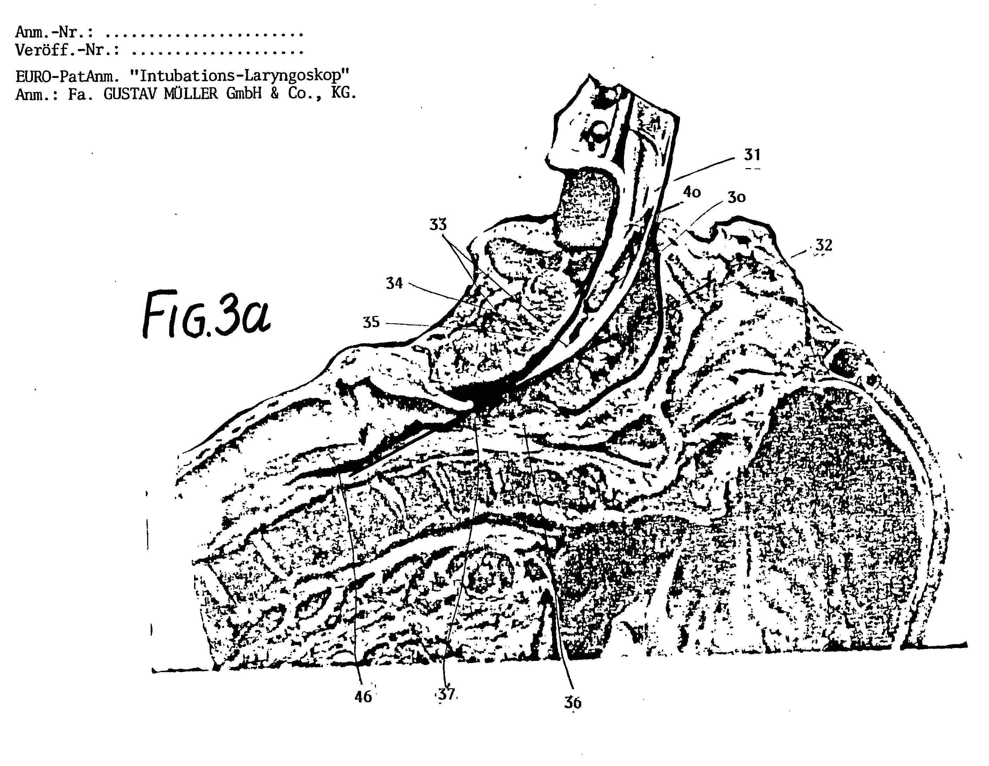

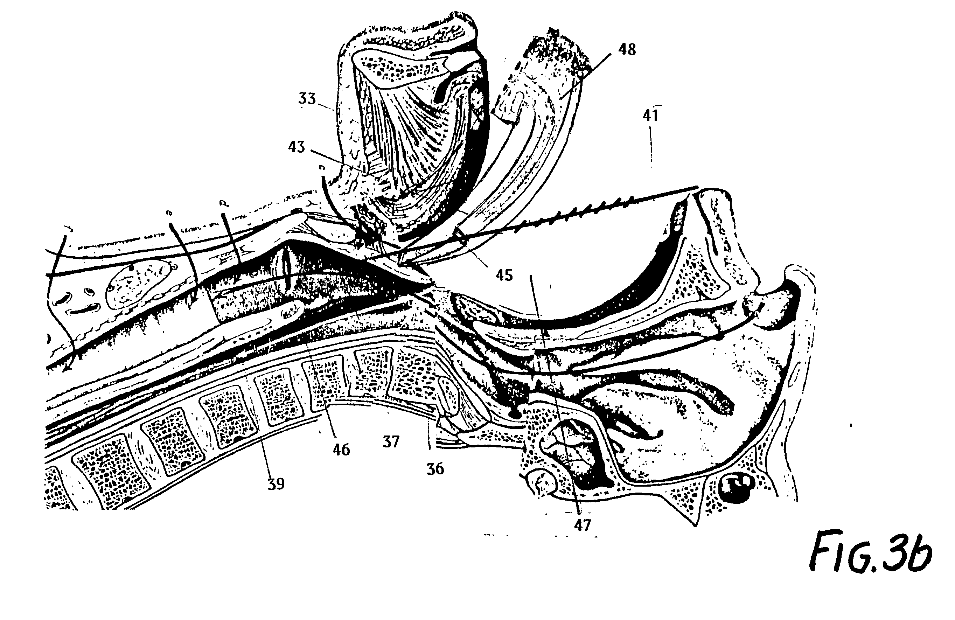

In Fig.3a ist der Vorgang der Vorbereitung einer Intubation dargestellt, wobei ein Warmlichtspatel 31 nach Macintosh mit Glühlampe 3o an der Seitenwand 4o des Spatel die Mundhöhle 32 eingeführt und die Zunge 33 durch das Spatelblatt 34 deutlich so zusammengepreßt ist, daß und um zusammen mit der speziellen Kopfhaltung einen geraden Lichtweg von dem höchsten Punkt 35 der zusammengepreßten Zunge, bzw. des Spatelblatts 34 über die trachea 36 nach dem Aufrichten der epiglottis 37 bis zum Kehlkopfeingang 39 herzustellen (ist).

In entsprechender Weise ist die in Fig.3b bei der erhöhten Lagerung und Reklination des Kopfes einerseits und durch Anheben des Zungengrundes 44 und der epiglottis 37 zur optimalen Intubation nötige Sicht auf die freien Stimmbänder 46 gewährleistet. Der gerade Lichtweg entlang der Tangente 41 vom höchsten Punkt 43, also der Zone des hinteren Zungenbereichs mit der größten Zungenmuskel-Masse der zusammengepreßten Zunge über die trachea 36 nach dem Aufrichten der epiglottis 37 bis zu den Stimmbändern 4o entspricht wiederum dem Fall der Fig.3a.

Die Lichtquelle 42 des Kaltlicht-Laryngoskops, dh. die zugehörige Lichtaustrittsöffnung 42 sitzt an der Stelle des höchsten Punktes 43 der Zunge im zusammengepreßten Zustand, ist definiert durch die Tangente 41 an die genannte Stelle 45 des Laryngoskops und liefert so die bereits erwähnte Verbindungslinie letzten Endes mit den Stimmbändern 46.

Nach dieser Maßnahme bzw. in diesem Behandlungszustand wird dann der Tubus über die Mundhöhle 32 bzw. 47 eingeführt. Wesentlich ist bei dieser Beschreibung der Zusammenhang, daß die Lichtquelle (Glühlampe) 30 bzw. Lichtaustrittsöffnung 42 an der Laryngoskop-Seitenwand 40 den Mundhöhlen-, trachea- und epiglottis-Kanal ausreichend gut ausleuchtet, wobei maßgebend ist, daß der Punkt der Lichtquelle hoch genug liegt, dh. oberhalb des foramen caecum, des Bereichs 43 der größten Dicke der Zunge, auch im Zustand der maximalen Pressung derselben.

In Fig .4 sind die Verhältnisse a) oben, ohne Laryngoskop und mit Ruhelage der Zunge 33 und b), unten mit eingeführtem Laryngoskop 48 und zusammengepreßter Zunge 34 stilisiert dargestellt. Im Vergleich ist der Lichtstrahl im oberen Bild als behindert und im unteren als frei durchgehend erkennbar. Der Sehstrahl kommt also im unteren Bild zum Auge der behandelnden Person am Laryngoskop 48 vorbei von den Stimmbändern 46 und aus trachea 36. Hierfür ist als Konstruktionshilfe die Tangente 51 an das Spatelblatt 35 über die trachea 36 bis zur epiglottis 37 und den Kehlkopfeingang 39 dienlich.

In Fig.5a) ist ein Spatel 3 mit Spatelblatt 61 mit eingesetztem Lichtleiter 62 in Gesamtansicht wiedergegeben. Die Bohrung 63 gehört zu der Schwenklagerung in dem nicht dargestellten Sockel, während die strichpunktierte Linie 64 ein Hinweis darauf ist, daß hier der Lichtfaser-Leitungsstrang gefaßt ist und in der ausgeschwenkten Lage in der Lichtbrücke zu der Lichtquelle liegt; diese Übergangsstelle ist in der Außenansicht b) durch die Stufenfassung 65 erkennbar.

Der Lichtfaser-Leitungsstrang ist mit einer Hilfsgehäusepackung 66 zusammengefaßt, die zum einen als stufenförmige Verdickung 67 ausgebildet in einem Durchbruch 68 in der Längsnut 69 aufgenommen und zum anderen unter Preßpassung in dem gekrümmten Ende 7o der Längsnut eingefügt ist; in diesem ist das Hilfsgehäuse etwas länger als die Längsnut im Spatelblatt, dient also der Lichtfaser-Leitungsstrang 62 für die Spannpressung durch Einklemmung zwischen dem Spann-Schnapp-Ende des einen Endes des Lichtleiters und der Schnappnase 69 am anderen Ende.

Fig.5c zeigt das Spatelblatt von oben, wobei der Durchbruch 68 von oben erkennbar ist, zusammen mit der abgeschrägt auslaufenden Abflachung 71, die eine Lichtführung für den Lichtaustritt bildet. Die an der Unterseite des Spatelblatts herausgearbeitete Längsnut 72 ist gestrichelt dargestellt, zusammen mit der ebenfalls gestrichelt dargestellten Führung 73 (Abschlußhülse 24) des Lichtleiters zu der Übergangsstelle im Handgriff (nicht dargestellt).

Die Außenkontur 74 verläuft im ganzen zungenförmig und im flachgekrümmten Anfangsteil 75 ab der Pharinx der Zunge (76) stets zunimmt bis zum Bereich 78 der größten Dicke der Zunge, wo die Breite etwa zwei Drittel der Mundhöhle einnimmt und von da an spitz gerundet entsprechend der Zungenspitze 77 ausläuft. Die Außenkontur 74 ist im übrigen symmetrisch zu der Längsachse und blattförmig.

Die Draufsicht auf die Hilfsgehäusepackung 66 ist in Fig.5d zusammen mit der stufenförmigen Verdickung 67 dargestellt. Fig.5e zeigt die Seitenansicht mit der Schnittlinie A-B der Schnittansicht der Fig.5a, wobei die Details der Schnappbefestigung in Fig.7 dargestellt sind.

In Fig .6 ist dargestellt, daß der Lichtleiter, der in einem Hilfsgehäuse 1o1 luft- und wasserdicht verschlossen untergebracht ist, in das Spatelblatt 1o2 von der Stirnseite 1o3 eingesteckt und dann in Richtung des Sockels 1o5 des Spatels zum Einschnappen bringt. Der Spatel hat im übrigen eine Lagerung durch Kugeln 1o6 (vgl. weiter unten) für die Schnappbefestigung, die in Seitenbacken der Halterungsvorrichtung des Handgriffs angeordnet ist.

Die eigentliche Lagerung des Spatels 3 mit ihrem Sockel in der Halterungsvorrichtung des Handgriffs ist bewirkt durch eine Kugelanordnung 1o7, ebenfalls in der Halterungsvorrichtung 1o8 des Handgriffs, indem die Kugelvorrichtung 1o7 in den halb offenen Lagerschlitz 1o9 gesteckt wird, an deren einer Seitenwand ein Nocken 11o angebracht ist, der infolge seiner Elastizität die Kugelvorrichtung 1o7 durchschnappen läßt und nach dem Durchlaß durch Rastung sichert. In dieser rastenden Lage ist der Lichtleiter mit seinem Hilfsgehäuse 1o1 durch den Haltezapfen 111 im Spatelsockel 112 gehalten.

Die Halterungsvorrichtung 1o8 (bzw. 6) wird nach ihrer Einführung in den Lagerschlitz 1o9 bei der Schwenkung in die Arbeitslage durch das Einschnappen der Kugeln 1o6 in die zugehörige Schnappvorrichtung 113 arretiert. Der Lichtleiter-Faserstrang ist also leicht und bequem zur Desinfektion und zum Ersatz auszutauschen.

In den Fig.7 a) bis c) sind Konstruktionsteile für den Lagerschlitz 1o9 wiedergegeben; a) in Unteransicht, b) in Seitenansicht, teilweise geschnitten, und c) in Rückansicht, teilweise ebenfalls im Schnitt. Der Lagerschlitz 1o9 des Spatelblatts mit den Lagerkugeln 1o6 zusammen mit den anderen nicht näher bezeichneten Details sind ohne weitere Erläuterungen verständlich. In Fig.7 d) ist die Lagerung der Kugeln in alternativer Art ebenfalls ohne weitere Erläuterungen verständlich dargestellt.

## Ansprüche

1. Gerät für ärztliche und/oder pflegerische Maßnahmen, insbesondere im Bereich von Operationsmethoden, wie der endotrachealen Intubation in der Anästhesiologie, Intensivtherapie und Notfallmedizin, im Zusammenhang mit visueller Untersuchung von Körperhöhlen und -gängen, bzw. im Bereich der Bronchial- und der Mund-Toilette, sowohl auf dem oralen bzw. dem nasalen Wege, mit einem Handgriff (1), an dessen Gehäuse, enthaltend eine elektrische Lichtquelle, eine zugehörige Energiequelle zu deren Stromversorgung und einen der Lichtquelle zugewandten Sockel (5), eine Halterungsvorrichtung (6) für ein darin griffstückseitig rastend schwenkbar gelagertes Formteil, zB. ein Spatel (15), mit Lichtkanal und dessen Lichtaustrittsöffnung (14, 42) nahe dem distalen zweiten Viertel seiner Länge angeordnet ist, dadurch gekennzeichnet, daß der Spatel (15) blattförmig und sonst in Längsrichtung in an sich bekannter Weise zungenförmig gestaltet ist, wobei seine Breite ab der Pharynx der Zunge bis zu ihrem foramen caecum, dem Bereich (76) der größten Dicke der Zunge, stetig zunimmt, an dieser Stelle mindestens annähernd zwei Drittel der Gaumenbreite einnimmt und von da ab in die gerundete Zungenspitze (77) ausläuft, während das Spatelblatt einen Längsabschnitt mit einstückigem, zueinander parallelen Längsstegen und -Nuten als Lichtkanal (äle) in die Ober- bzw. Unterfläche herausgearbeitet aufweist, die im Extremfalle Längsdurchbrüche (zB. 2o, 68) bilden und in welche der Lichtleiter (62) bis zur Dicke des Spatelblatts (61) eingetaucht ist, wobei die Lichtaustrittsöffnung gegebenenfalls den Lichtkanal bzw. den Lichtleiter, zB. den Lichtleiter-Faserstrang (18), an der Stelle des höchsten Punktes 43, also der Zone des hinteren Zungenbereichs mit der größten Zungenmuskelmasse der zusammengepreßten Zunge, nahe dem Berührungspunkt der fiktiven Tangente (51) an die Unterfläche des Spatelblatts (11), von der trachea (36) aus angelegt, an der Stelle der medizinisch optimalen Pressung der Zunge während des Einsatzes des Laryngoskops, dh. mindestens annähernd in üblichem Abstand vom distalen Ende des Spatelblatts liegt.

2. Gerät nach Anspruch 1,
dadurch gekennzeichnet, daß das Spatelblatt (11) mindestens eine Längslücke (Längsrippe 19) und zwei Längs- dadurch gekennzeichnet,
daß der Spatel (15) blattförmig und sonst in Längsrichtung in an sich bekannter Weise zungenförmig gestaltet ist, wobei seine Breite ab der Pharynx der Zunge bis zu ihrem foramen caecum, dem Bereich (76) der größten Dicke der Zunge, stetig zunimmt, an dieser Stelle mindestens annähernd zwei Drittel der Gaumenbreite einnimmt und von da ab in die gerundete Zungenspitze (77) ausläuft,
während das Spatelblatt einen Längsabschnitt mit einstückigem, zueinander parallelen Längsstegen und -Nuten als Lichtkanal (äle) in die Ober- bzw. Unterfläche herausgearbeitet aufweist, die im Extremfalle Längsdurchbrüche (zB. 2o, 68) bilden und in welche der Lichtleiter (62) bis zur Dicke des Spatelblatts (61) eingetaucht ist, wobei die Lichtaustrittsöffnung gegebenenfalls den Lichtkanal bzw. den Lichtleiter, zB. den Lichtleiter-Faserstrang (18), nach der Zungenspitze (77) hin abschließt und das Ende des Lichtleiters, dh. die Lichtaustrittsöffnung, nahe dem Berührungspunkt der fiktiven Tangente (51) an die Oberfläche des Spatels (3), von der trachea (36) aus angelegt, in der Lage der medizinisch optimalen Pressung der Zunge während des Einsatzes des Laryngoskops liegt.

2. Gerät nach Anspruch 1,
dadurch gekennzeichnet, daß das Spatelblatt (11) mindestens eine Längslücke (Längsrippe 19) und zwei Längs stege zu (mindestens) einem mittigen Lichtkanal für einen gemeinsamen Lichtfaserstrang (18) mit einer gemeinsamen Lichtaustrittsöffnung (14) zusammengefaßt ist, welche mit einer Abschrägung zum Übergang entsprechend dar Zungenspitze (77) verläuft.

3. Gerät nach einem der Ansprüche 1 und 2,
dadurch gekennzeichnet, daß der Lichtleiter gegebenenfalls einschließlich Hilfsgehäuse (1o1) in dem Lichtkanal als Formkörper angepaßt ortsfest zwischen Sockel (1o5) und Lichtaustritts-Öffnung (14) eingefügt ist.

4. Gerät für ärztliche und/oder pflegerische Maßnahme, insbesondere im Bereich von Operationsmethoden, wie der endotrachealen Intubation in der Anästhesiologie, Intensivtherapie und Notfallmedizin, im Zusammenhang mit visueller Untersuchung von Körperhöhlen und -gängen, bzw. im Bereich der Bronchial- und der Mund-Toilette, sowohl auf dem oralen bzw. dem nasalen Wege,
mit einem Handgriff (1), an dessen Gehäuse, enthaltend eine elektrische Lichtquelle, eine zugehörige Energiequelle zu deren Stromversorgung und einen der Lichtquelle zugewandten Sockel (5), eine Halterungsvorrichtung (6) für ein darin griffstückseitig rastend schwenkbar gelagertes Formteil, zB. ein Spatel (15), mit lichtkanal und dessen Lichtaustrittsöffnung (14, 42) nahe dem distalen zweiten Viertel seiner Länge angeordnet ist,
wobei das Formteil (zB. der Spatel 3) mindestens in einem Teil seiner Länge einen Profil-förmigen Querschnitt aufweist, indem an den Seitenrändern des Mittelstegs ein erster im rechten Winkel dazu abgebogener schmalerer (23) und ein zweiter entgegengesetzt gebogener breiterer Schenkel (24) angeformt sind, von mit Ausnahme einer Längsrille im breiteren Schenkel parallel zum Mittelsteg durchgehend gleicher Wandstärke und an einer der Seitenwände des Mittelstegs ein L-Profilstück mit seinem längeren Schenkel im Abstand des kürzeren Schenkels von dem Mittelsteg angeordnet enthält, wobei dieser kürzere Schenkel des 1.Profilstücks mit der Kante des schmaleren Schenkels des 2.Profilstücks bündig abschließt und somit oberflächlich-homogen lückenfrei verschlossen einstückig vereinigbar ist,
während das Spatelblatt einen Längsabschnitt mit einstückigem, zueinander parallelen Längsstegen und -Nuten als Lichtkanal (äle) in die Ober- bzw. Unterfläche herausgearbeitet aufweist, die im Extremfalle Längsdurchbrüche (zB. 2o, 68) bilden und in welche der Lichtleiter (62) bis zur Dicke des Spatelblatts (61) eingetaucht ist,
dadurch gekennzeichnet, daß der Lichtleiter in einem an den Lichtkanal als Längsrippe (19) angepaßten, dichtverschlossenen Formgehäuse (7o) mit Schnappbefestigung (113) durch eine Schnappvorrichtung arretierbar und austauschbar untergebracht ist.

5. Gerät nach einem der Ansprüche 1 bis,
dadurch gekennzeichnet, daß die Längsrippe (19) in einem zwischen Sockel (5) und Lichtaustritts-Öffnung (14) in dem Spatelblatt (61) ausgebrochenen, vorzugsweise mittigen durchgehenden Schlitz einer Längsnut (2o) versenkt ist.

6. Gerät nach Anspruch 5,
dadurch gekennzeichnet, daß die Längsrippe (19) formschlüssig unter Preßpassung zwischen einem Anschlag am Sockelende (1o5) und dem Anschlag am Lichtaustrittsöffnungs-Ende (14) infolge Krümmungsverspannung mit Hilfe einer elastischen Nok-
kenanordnung (114) arretiert ist.

Anm.-Nr.: ...............
Veröff.-Nr.: ............

EURO-PatAnm. "Intubation;
Anm.: Fa. GUSTAV MÜLLER

24·04·88

FIG.1

Anm.-Nr.: ...................
Veröff.-Nr.: ...............

EURO-PatAnm. "Intubations-La
Anm.: Fa. GUSTAV MÜLLER GmbF

FIG. 2

Anm.-Nr.: ........................
Veröff.-Nr.: .....................

EURO-PatAnm. "Intubations-Laryngoskop"
Anm.: Fa. GUSTAV MÜLLER GmbH & Co., KG.

FIG.3a

EP 0 339 541 A1

Anm.-Nr.: ........................
Veröff.-Nr.: ....................

EURO-PatAnm. "Intubations-Laryngoskop"
Anm.: Fa. GUSTAV MÜLLER GmbH & Co., KG.

FIG.3b

EP 0 339 541 A1

Anm.-Nr.: ..............
Veröff.-Nr.: ...........

EURO-PatAnm. "Intubations-Laryngoskop"
Anm.: Fa. GUSTAV MÜLLER GmbH & Co., KG.

36,37

# FIG.4

36,37

Anm.-Nr.: ..............
Veröff.-Nr.: ...........
EURO-PatAnm. "Intubation:
Anm.: Fa. GUSTAV MÜLLER (

A-B    68   69   71

7o

61

62

a

114

63

64

67

66

3

b

A

114

65

65

24

B

e

74

76

c

75

77

73

72

66      67

FIG.5      d

FIG.6

*a*

112

*c*

*b*

1o7

114

1o9

*C-D*     *d*

.1o6

FIG.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-3 766 909 (A. M. OZBEY) <br> * Spalte 6, Zeilen 2-33; Figuren 1,2 * | 1,2 | A 61 B 1/26 |
| A | | 3-5 | |
| | --- | | |
| A | DE-A-2 728 910 (J. R. BULLARD) <br> * Seite 10, Zeile 11 - Seite 12, Zeile 28; Figuren 1-4 * | 1-6 | |
| | --- | | |
| A | GB-A-2 105 994 (P. MURPHY) <br> * Seite 2, Zeile 37 - Seite 3, Zeile 7; Figuren 1,2,5 * | 1,4 | |
| | ----- | | |

**RECHERCHIERTE SACHGEBIETE (Int. Cl.4)**

A 61 B 1/00

Der vorliegende Recherchenbericht wurde für alle Patentansprüche erstellt

| Recherchenort | Abschlußdatum der Recherche | Prüfer |
|---|---|---|
| BERLIN | 18-07-1989 | WEIHS J.A. |